# EUROPEAN PATENT APPLICATION

(11) **EP 2 074 975 A2**
(43) Date of publication of application: **01.07.2009**
(21) Application number: 09003864.7
(22) Date of filing: 29.09.2004
(51) Int. Cl.: A61F 13/15

(54) **Customizable absorbent article with extensible layers**

(30) Priority: 30.12.2003 US 750404
(62) Divisional of application: 04789529.7
(71) Applicant: KIMBERLY-CLARK WORLDWIDE, INC., Neenah, WI 54956 (US)
(72) Inventor: Price, Cindy, Appleton WI 54913 (US); Dipalma, Joseph, Neenah WI 54956 (US); Wallajapet, Palani Raj, Neenah WI 54956 (US); Mocaldo, Cheryl, New London WI 54961 (US); Weinheimer, Amy, Appleton WI 54915 (US)
(74) Representative: Davies, Christopher Robert

(57) **Abstract**

The invention includes a customizable absorbent article having an extensible body facing layer and an extensible garment facing layer, both having a first end and a second end. The first and second ends are spaced along a longitudinal axis and the body facing and garment facing layers are extensible along the longitudinal axis. The article further includes an absorbent core which is disposed between the body facing layer and the garment facing layer. The absorbent core may be affixed to at least one of the body facing and the garment facing layer at at least one location between the first and second ends. Different configurations of the absorbent core may be disposed between the body facing layer and the garment facing layer to increase the customizability of the absorbent article of the present invention.

## Description

### BACKGROUND

The present invention relates generally to absorbent articles for absorbing and retaining human exudates, and in particular, to an absorbent article that is extensible, allowing the user to customize the length, width, and or thickness of the article with substantially sustained deformation of the garment facing layer, the body facing layer, and/or the absorbent core.

Absorbent articles such as sanitary napkins, incontinent garments, incontinent shields, and the like are designed to be worn as part of an absorbent garment or independently in a user's undergarment and adjacent a user's body to absorb body fluids such as menses, blood, urine, and other excrements. Conventional absorbent articles have pre-determined thicknesses or shapes. However, many conventional absorbent articles do not function well with the variety of users' bodies or needs. Thus, a consumer must purchase a variety of different articles for the various needs throughout, for example, an individual's menstrual cycle. An article that may be suitable for day time wear may not be suitable for night time wear due to the changes in body positioning, direction of fluid flow and the like. Therefore, it would be desirable to have an absorbent article that could be adjusted or customized for the individual user's needs.

In addition, conventional incontinent shields and sanitary napkins may not function well with the variety of user's undergarments. For example, conventional articles may not stretch out with the user's undergarments as they are being pulled up into place or during body movement. An absorbent article that could be stretched to a point of sustained deformation by the consumer can therefore be customized to fit a particular consumer's underwear and body. Accordingly, conventional articles may not provide the desired levels of fit, absorbency and comfort. The present invention is an absorbent article that will remedy these, and other, problems of the prior endeavors. These attributes will become clear as the present invention is more thoroughly discussed in this application.

### SUMMARY

Briefly stated, in one aspect, the invention includes a customizable absorbent article having an extensible body facing layer and an extensible garment facing layer, both having a first end and a second end. The first and second ends are spaced along a longitudinal axis and the body'facing and garment facing layers are extensible. Preferably, the body facing and garment facing layers are extensible along the longitudinal axis, but may also be extensible along a lateral axis or in a Z direction. The article further includes a non-extensible absorbent core which is disposed between the body facing layer and the garment facing layer. The absorbent core may be affixed to at least one of the body facing and the garment facing layer at at least one location between the first and second ends.

In another aspect, the article of the present invention includes an extensible body facing layer and an extensible garment facing layer, both having a first end and a second end spaced along a longitudinal axis. The article further includes an absorbent core that is permanently deformable, the absorbent core being manually movable from a pre-use condition, wherein the absorbent core has a first thickness, to an in-use condition, wherein the absorbent core has a second thickness that is different than the first thickness of the absorbent core in the pre-use condition. The absorbent core may be minimally affixed to at least one of the body facing layer and the garment facing layer.

In yet another aspect, a customizable absorbent article includes an extensible body facing layer and an extensible garment facing layer both having a first end and a second end, the first and second ends being spaced along a longitudinal axis. Preferably, the body facing and garment facing layers are extensible along the longitudinal axis, but may also be extensible along a lateral axis or in a Z direction. The article further includes a first absorbent layer having a first portion and a second portion, the first and second portions being movable relative to each other from a pre-use condition, wherein the first portion and the second portions are approximately adjacent one another, to an in-use condition, wherein the first portion and the second portion are separated from one another by a distance. The article also includes an absorbent core, a portion of which is exposed to at least one of the body facing layer and the garment facing layer when the first and second portions of the absorbent layer are moved to the in-use condition.

The present invention also includes a method for absorbing bodily exudates with a customizable absorbent article. The method includes the step of providing a customizable absorbent article comprising an extensible body facing layer and an extensible garment facing layer, both having a first end and a second end spaced along a longitudinal axis and both being extensible along the longitudinal axis. The absorbent article also includes a non-extensible absorbent core disposed between the body facing layer and the garment facing layer and may be affixed to at least one of the body facing and the garment facing layer at at least one location between the first and second ends. The method further includes manually adjusting the customizable absorbent article along the longitudinal axis by applying force along the axis, thereby deforming the extensible absorbent body facing layer and garment facing layer. The method further includes securing the absorbent article to an undergarment using a releasable attachment portion.

### BRIEF DESCRIPTION OF THE DRAWINGS

FIG. 1 is a top perspective view of one aspect of the present invention;
FIG. 2 is a cross sectional view of one aspect of an article of the present invention;
FIG. 3 is cross sectional view of another aspect of an article of the present invention;
FIG. 4A is a top view of one aspect of an absorbent core of the present invention in pre-use condition;
FIG. 4B is a top view of the core of FIG. 4A in an in-use condition;
FIG. 5 is a top perspective view of an embodiment of an absorbent article of the present invention.
FIG. 5A is a cross sectional view of one embodiment of an absorbent core of the absorbent article of FIG. 5 in pre-use condition, taken along line 5A-5A;
FIG. 5B is a cross sectional view of the core of FIG. 5A in an in-use condition;
FIG. 5C is a cross sectional view of one embodiment of an absorbent core of the absorbent article of FIG. 5 in pre-use condition, taken along either line 5C-5C;
FIG. 5D is a cross sectional view of the core of FIG. 5C in an in-use condition;
FIGS. 6A, 6B, and 6C are schematic representations of an absorbent core of the present invention in first pre-use, second pre-use, and in-use conditions, respectively;
FIGS. 7A and 7B are schematic representations of an absorbent core of the present invention in pre-use and in-use conditions, respectively;
FIGS. 8A, 8B, and 8C are schematic representations of an absorbent core of the present invention in first pre-use, second pre-use, and in-use conditions, respectively;
FIG. 9 is a bottom view of one aspect of the present invention; and
FIG. 10 is a bottom view of another aspect of the present invention.

### DETAILED DESCRIPTION OF THE INVENTION

As used herein, the term "airlaid" refers to the process of producing an absorbent material where unlike components are conveyed in an air-stream and homogenously mixed or provided in a stratified configuration and then bonded together. For example, this may include, but is not limited to, the mixture of pulp fibers, synthetic fibers, superabsorbent materials and binder material. The binder material is often, but not limited to, synthetic bicomponent binder fibers and or latexes. There are a number of commercial processes available to produce airlaid absorbent structures. For example; airlaid processes are available from Danweb Corp. having offices in Risskov, Denmark, and from M&J Forming Technologies having offices in Horsens, Denmark. Examples of suitable products and the process for forming them are described in U.S. Pat. No. 4,640,810 to Laursen et. al., U.S. Pat. No. 4,494,278 to Kroyer et. al., U.S. Pat. No. 4,351,793 to Day, and U.S. Pat. No. 4,264,289 to Day, the relevant portions of which are incorporated by reference.

An airlaid process provides a mixture of raw materials and the ability to add synthetic fibers and/or binder agents to the mixture to stabilize the resultant absorbent. As a stabilizer, binders reduce the amount of wet collapse in the structure and maintain a lower density in the saturated state. That is, the binder assists the absorbent matrix in maintaining its integrity even under load or while saturated. In addition, the resulting structure has both a higher dry and wet tensile strength than a corresponding structure without a binding agent.

The term "body-facing" should not be interpreted to mean in contact with the body of the user, but rather simply means the side that would face toward the body of the user, regardless of whether an undergarment is actually being worn by the user and regardless of whether there are or may be intervening layers between the component and the body of the user. Likewise, the term "garment-facing" should not be interpreted to mean in contact with the garments of the user, but rather simply means the side that faces away from the body of the user, and therefore toward any outer garments that may be worn by the user; regardless of whether the undergarment is actually being worn by a user, regardless of whether any such outer garments are actually worn and regardless of whether there may be intervening layers between the component and any outer garment.

As used herein, the "crotch region" of an absorbent article refers to the generally central region that will be in contact with the crotch of a user, near the lowermost part of the torso, and resides between the front and rear portions of the article. Typically, the crotch region generally extends in the longitudinal direction depending on the function of the absorbent article.

As used herein, the term "customizable" refers to an article or material that the user can alter, after purchase to better suit their needs. For example, if a user wishes to increase the length of an absorbent article for use at night, the article may be customized to meet this wish. If the user desires more absorbent material in one area or another of the absorbent article, the user may adjust the thickness or bulk of the absorbent material to meet this need. An article may be customized through manual deformation by the user or through the natural range of motion experienced by the material during use of the article.

As used here, the term "extensible" refers to a material that is capable of elongation when subjected to an applied tensile force. The material also is preferably capable of providing a selected, sustained deformation when subjected to an applied tensile force and-then allowed to relax after removal of the tensile force. Extensible materials are preferably capable of retracting minimally or not at all, due in part to the condition of hysteresis, along the X, Y, or Z axis of the absorbent article, when subjected to an applied tensile force and then allowed to relax after removal of the tensile force. Preferably the sustained deformation is substantially permanent deformation. The selected elongation and sustained deformation preferably occur at least along the longitudinal cross-direction of the material, although it should be understood that it also could occur along the lateral direction, the Z direction or all three directions.

It should be understood that the term "longitudinal," as used herein, means of or relating to the length or the lengthwise direction. The term "laterally," as used herein, means situated on, directed toward or running from side to side in a direction substantially perpendicular to the lengthwise direction. The term "lateral" or "laterally", as used herein, may also include movement in the X-Y plane.

The term "pledget" refers to an absorbent layer that has a length, width and/or thickness that is less than the length, width, and/or thickness of at least one of the layers to which it is adjacent.

The phrase "pre-use" refers to the state of the absorbent article before the user has customized the article to fit his or her individual needs. The phrase "in-use" refers to the state of the absorbent article after the user has adjusted the dimensions of the absorbent article to his or her desired configuration.

The phrase "releasable" refers to the characteristic of one or more elements being, securely but not permanently, affixed to one another. The required separation force is typically beyond that encountered through normal wear of an absorbent article in the undergarment of the user.

The term "superabsorbent" refers to a water-swellable, water-insoluble, organic or inorganic material capable, under the most favorable conditions, of absorbing at least about 15 times its weight and, more desirably, at least about 30 times its weight of an aqueous solution containing 0.9 weight percent sodium chloride. The super absorbent materials can be natural, synthetic, and modified natural polymers and materials. In addition, the super absorbent materials can be inorganic materials, such as silica gels, or organic compounds such as cross-linked polymers. Examples of superabsorbents are disclosed in U.S. Patent No. 5,609,588 to DiPalma et. al., column 8, line 1 to line 57, which portion is incorporated herein by reference. A further example of a superabsorbent material is FAVOR SXM-880 available from Stockhausen Inc. 2401 Doyle Street, Greensboro, NC 27406, USA.

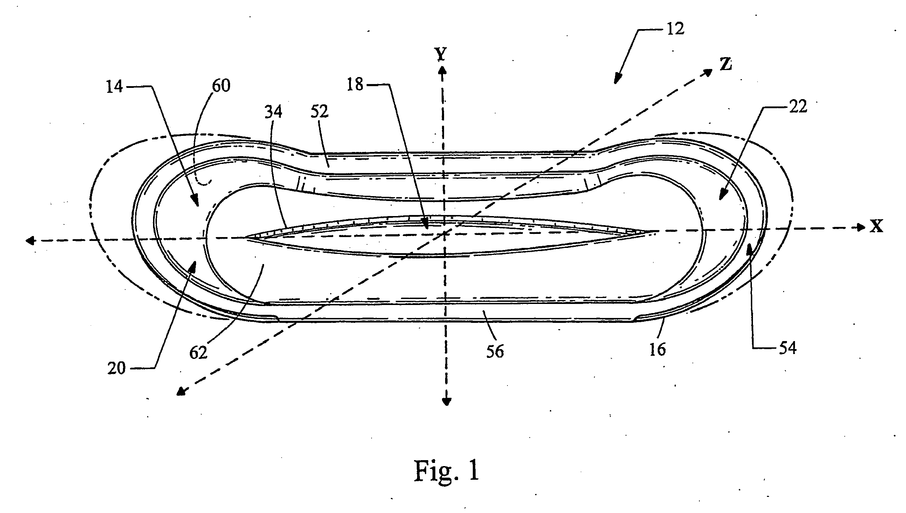

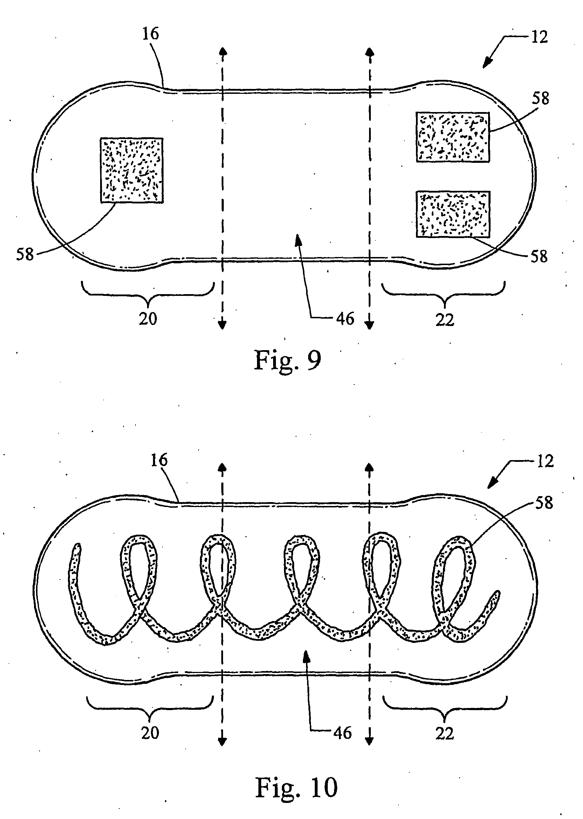

Referring now to FIG. 1, the absorbent article 12 of the present invention is customizable to the users needs. The article 12 includes a body facing layer 14, a garment facing layer 16 (See FIGS. 9 and 10) and an absorbent core 18. The body facing layer 14 is designed to contact the body of the user and desirably is liquid-permeable. The garment facing layer 16 is generally liquid-impermeable and is designed to face the inner surface, i.e. the crotch region, of the user's underwear (not shown). The body facing layer 14 and the garment facing layer 16 have first and second ends 20 and 22 and are desirably extensible along a longitudinal'axis, X, but may also be extensible along a lateral axis, Y. As shown in FIG. 1, the body facing 14 and the garment facing layers 16 are manually movable from a pre-use condition to an in-use condition. The user may extend the body facing 14 and garment facing layers 16 along the longitudinal axis X to adjust the length of the article 12 to a desired configuration. The amount of applied pressure needed to extend the layers is only that sufficient to move the layers 14 and 16 into the in-use condition. For example, the body facing layer 14 and the garment facing layer 16 are extensible in that they are capable of providing an elongation of at least about 1 cm when subjected to a tensile force of 11.8 grams per centimeter (g/cm), and further provide a substantially permanent deformation of at least about 20% when subjected to a tensile force of 19.70 g/cm and is then allowed to relax under a zero applied stress for a period of 1 minute. The length (cm) is measured along the test sample, perpendicular to the applied tensile force.

Various extensible body facing materials include necked or creped spunbond and sheath/core polypropylene/KRATON elastomeric bicomponent spunbond, described in U.S. application Serial Number 10/647,008 to Rhim et al. and U.S. Patent No. 6,150,002 to Eugenio Go Verona. Various extensible garment facing materials are described in U.S. Patent No. 6,096,014 to Haffner et al. (extensible biaxial film). Other suitable materials for use as the body facing and garment facing layers may include materials composed (entirely or partially) of elastomeric polymers that impart extensibility to the web, or in the case of nonextensible materials, such as nonwoven webs, laminates, spunbond, meltblown or bonded-carded web composed of synthetic polymer filaments or fibers, such as polypropylene, polyethylene, polyesters, or the like, perforated films, webs of natural polymer filaments or fibers such as rayon or cotton, be constructed by such a means as to allow for extensibility of the layer.

The body facing layer 14 may include at least one aperture 34 disposed in approximately the center of the body facing layer 14. This aperture 34 allows insults of fluid or body menses to directly contact the absorbent core 18 which is desirably disposed between the body facing 14 and garment facing layer 16. In this execution, the body facing layer 14 may be partially or entirely hydrophobic.

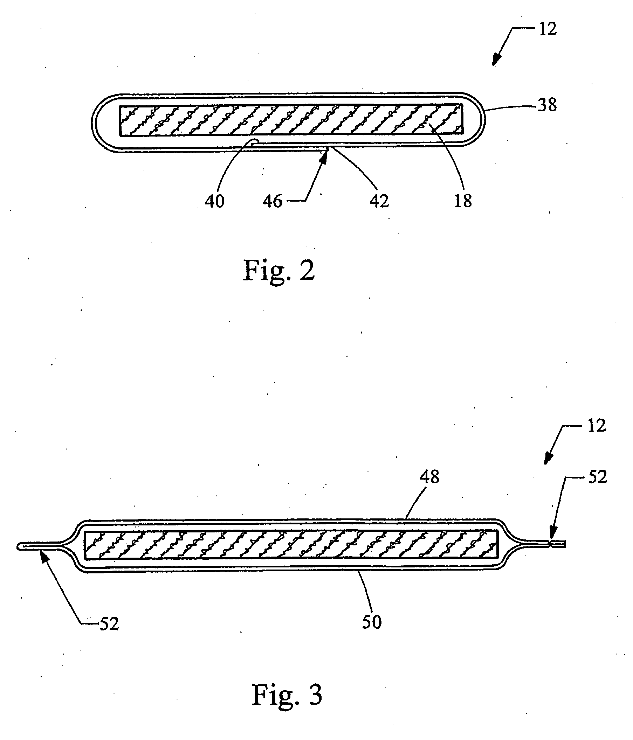

Also, the body facing layer 14 and the garment facing layer 16 may be comprised of one or more individual sheets of extensible material. For example, with reference to FIG. 2, the body facing and garment facing layers may be made of one continuous sheet of extensible material 38. The sheet 38 is wrapped around the absorbent core 18 and opposite ends 40 and 42 of the sheet 38 are affixed to one another at a predetermined location about the article 12. Preferably, the point at which the opposed ends 40 and 42 of the extensible sheet 38 are affixed is located toward the garment facing surface 46 of the absorbent article 12, but may be located at any point on the article 12. If the body facing and garment facing layers are comprised of one individual sheet 38, it is preferable that the material be wrapped about the absorbent core 18 in such a manner as to allow the sheet 38 to extend along the longitudinal axis X. If the core is wrapped in one fluid permeable extensible sheet, as shown, additional fluid impervious material (not shown) may be incorporated to prevent fluid passage to the underwear and outer garments. An example of such a material would be a low gauge polyethylene film. Desirable materials would likewise be extensible. Such materials include biaxial extensible film such as the polyethylene film is described in U.S. Patent No. 6,096,014 to Haffner et al. (see description of garment facing layer, above) and heavy adhesive spray, such as Dispomelt 2525A, which is commercially available from National Starch Corp., Bridgewater, New Jersey, or any other suitable elastomeric adhesive.

In the alternative, the body facing layer and the garment facing layer may be comprised of more than one individual sheet of extensible material, as shown in FIG. 3. As such, the absorbent core 18 will desirably be disposed between the sheets 48 and 50 of extensible material that define the body and garment facing layers. The sheets of extensible material 48 and 50 can be coextensive in a face-to-face contact around the outer edge of the absorbent core 18. The sheets 48 and 50 can be sealed together about their peripheries by use of an adhesive, by heat sealing, ultrasonics, embossing, or by any other process known in the art. A suitable hotmelt adhesive, used to seal the peripheries of the absorbent article is Dispomelt 2525A and is commercially available from the National Starch Corp., Bridgewater, New Jersey.

Referring again to FIG. 1, this sealed area is the peripheral seal 52. The peripheral seal 52 has end margins 54 and side margins 56. The length and width dimensions of the body facing layer 14 and the garment facing layer 16 are generally lager than and extend beyond the corresponding dimensions of the absorbent core 18 to provide for the corresponding end margins 54 and side margins 56. The shape, length, and width, of the absorbent article 12 will be defined by the peripheral edges of the body facing layer 14 and garment facing layer 16.

The absorbent article 12 of the present invention may be affixed to the undergarment of the user (not shown) by a variety of methods known to those of skill in the art. Desirably, the absorbent article 12 is extended to the user's desired length by applying pressure to the body facing layer 14 and the garment facing layer 16 along the longitudinal axis X. Once extended, the user may secure the article 12 to the crotch region of her underwear with a releasable attachment component 58 that is desirably disposed on the garment facing layer 16 of the absorbent article 12. The attachment component 58 is desirably disposed in discrete areas about the underside of the garment facing layer 16. More desirably, the attachment component 58 is disposed in discrete areas about the first 20 and second 22 end portions of the garment facing layer 16 as shown in FIG. 9. The placement of the attachment component 58 should not inhibit the extension of the garment facing layer 16.

Likewise, the attachment component 58 may be disposed about the garment facing layer 16 in a discreet or continuous pattern, such as circles, spirals, patches, or dashed lines, allowing the material of the garment facing layer 16 to stretch and extend uninhibited by the attachment component 58, as shown in FIG. 10. The attachment component may be stretchable adhesive or mechanical fasteners. Examples of suitable attachment components are described in U.S. Pat. No. 5,540,673 to Thomas, U.S. Pat. No. 5392498 to Goulait, and U.S. Pat. No. 6,489,004 to Martin.

Referring again to FIG. 1, the absorbent article 12 of the present invention includes an absorbent core 18. The absorbent core 18 is desirably disposed between the body facing 14 and the garment facing layers 16. The absorbent core 18 will include at least one layer, but as discussed below, the absorbent core 18 may include more than one layer in more than one configuration. Each layer may be comprised of the same or different materials. The absorbent core 18 provides an absorbent structure that is configured for holding and storing absorbed liquids and other waste materials. The absorbent core can be.composed of any material that will absorb bodily exudates such as menses, blood, and urine. Suitable absorbents include cellulose fluff pulp, wood fluff, rayon, cotton, superabsorbents, foam, and mixtures thereof. Additionally stabilized absorbents such as airlaid can be used. Meltblown polymers, such as polyester, polypropylene, or combinations thereof, hydroentangled pulp, tissue, and elastic scrim can also be used. Desirably, a thin and flexible absorbent material, such as elastic coform, can be used. Elastic coform can be made according to the process disclosed in U.S. Patent 6,362,389 to McDowall et al., col. 11, lines 66-67 which refers to U.S. Patents 4,818,464 to Lau and 4,100,324 to Anderson et. al., which are hereby incorporated by reference. Elastic scrim can be made according to the process disclosed in U.S. Patent Application Serial Number 10/620,142. Superabsorbents on stretchable substrate can be made according to the process disclosed in U.S. Patent Application Serial No. 10/699193 to Sawyer et al.

As shown in FIG. 1, the absorbent core 18 may include a first absorbent layer 60 and may further include a surge layer 62. Desirably the surge layer 62 is disposed between the body facing layer 14 and the first absorbent layer 60 and may be exposed to the body of the user through the aperture 34 in the body facing layer 14. Suitable materials for use as a surge layer include bonded carded webs including through-air bonded carded webs, bicomponent spunbond, and low basis weight, low density airlaids. Structures composed either entirely or in part of polyester fiber are particularly desirable as a means to impart resiliency and maintain an open, low density structure. Such materials are described in U.S. Patent No. 5,994,615 to Dodge et al. The absorbent core 18 may be affixed to either or both the body facing layer 14 and the garment facing layer 16 at a location between the first and second ends 20 and 22 of the body facing 14 and garment facing 16 layers. The core 18 may be secured to the outer layers 14 and 16 by any suitable method of attachment known in the art.

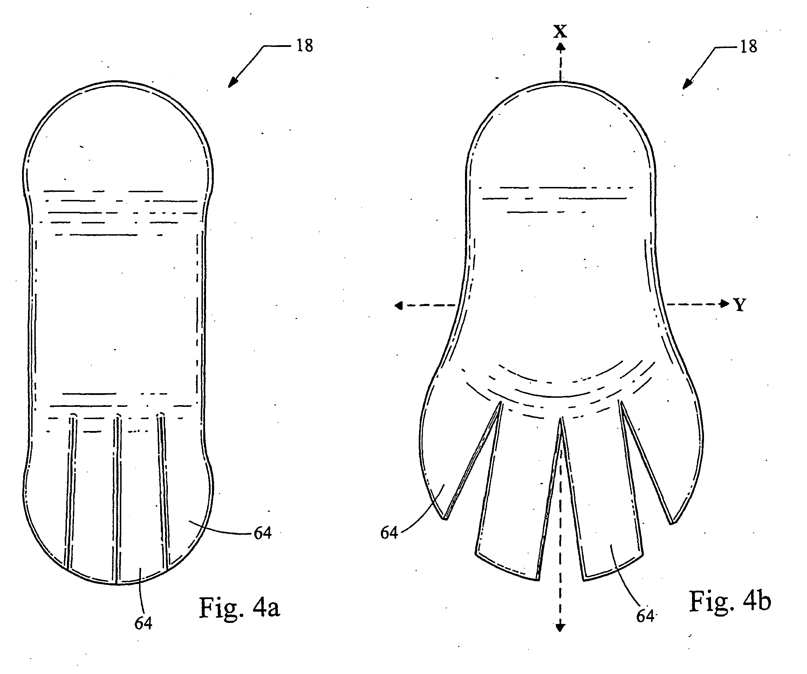

In another embodiment of the present invention, one or both ends of the absorbent core 18 may be designed to have individual members 64 which move, relative to one another, along a lateral axis Y. An absorbent core, made of non-extensible absorbent materials may be die-cut at one end, approximately along the longitudinal axis X, as shown in FIG. 4A. This cutting allows the user to pull the product laterally outward, stretching the extensible body and garment facing layers, while at the same time moving each individual member 64 in a "fan" shape as shown in FIG. 4B.,meltblown microfiber or wetlaid. Suitable absorbents for this embodiment include cellulose fluff, wood fluff, rayon, cotton, superabsorbents, foams, and mixtures thereof. Preferably, stabilized absorbents such as airlaid, coform or wetlaid absorbent can be used. Meltblown polymers, such as polyester, polypropylene, or combinations thereof, hydroentangled pulp and tissue can also be used, although fluff alone may be used. Such absorbent materials, such as coform and meltblown microfibers can be obtained from Kimberly-Clark, Incorporated, Neenah, WI and airlaid absorbent structures may be obtained from Concert Industries, 1680 rue Atmec, Gatineau, Quebec, Canada J8P 7G7.

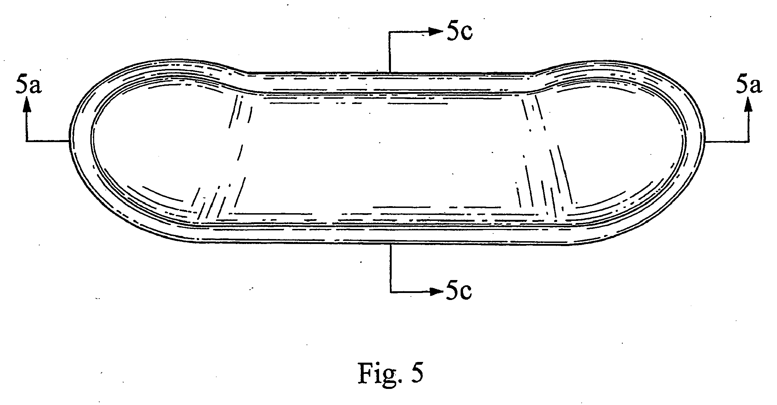

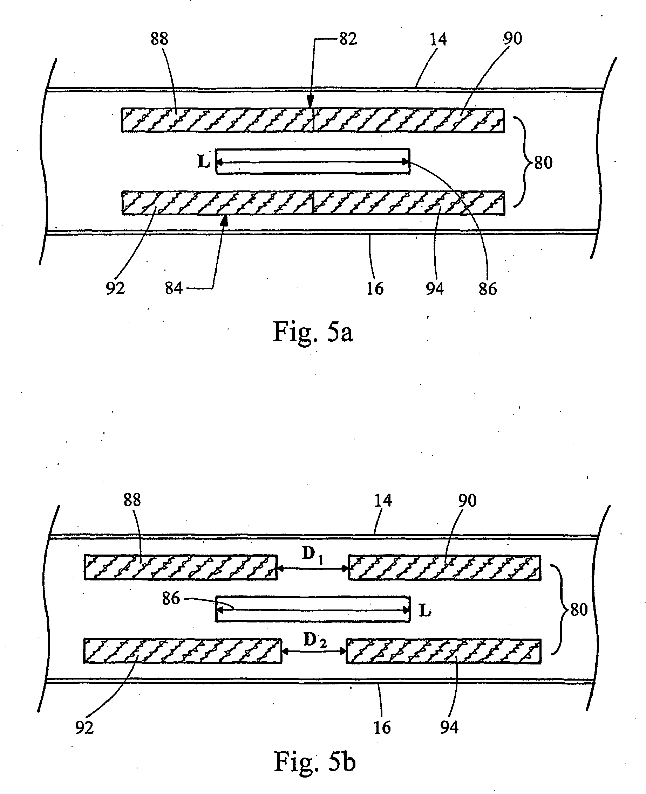

FIGS. 5A and 5B show yet another embodiment of an absorbent core of the present invention. By layering the core materials that are disposed between the extensible body facing and garment facing layers, the present invention creates a core that can be stretched in the longitudinal or lateral direction. The core may be customizable in the Z direction as well, depending on the properties of the materials used. The core materials are layered in a manner that allows them to slide across one another. The core components may be all the same material or different materials, depending on the core properties desired.

For example, an absorbent core 80 may include at least first absorbent layer 82 and an absorbent pledget 86. More desirably, the core 80 includes a first absorbent layer 82, an absorbent pledget 86, and an optional second absorbent layer 84. The optional second absorbent layer provides added absorbent capacity and use of it is dependent on the level of absorbency desired. The first and second absorbent layers 82 and 84 may both include a first portion 88 and 92 and a second portion 90 and 94 which are movable relative to each other from a pre-use to an in-use condition. As shown in FIG. 5A, in the pre-use condition, the first and second portions 88 and 90 of the first absorbent layer 82 and the first and second portions 92 and 94 of the second absorbent layer 84 are approximately adjacent one another. In an in-use condition, as shown in FIG. 5B, the first and second portions 88 and 90 move in opposite directions and are separated from one another by distances D₁ and D₂. Distances D₁ and D₂ are equal to or less than the pledget length or width and limited by the extensibility of the body and garment facing layers. This separation allows the absorbent layers 82 and 84 to move with an extensible body facing layer and garment facing layer when extended by the user.

The absorbent pledget 86 is desirably disposed between the first and second absorbent layers 82 and 84 and is typically no more than approximately three-fourths the length of the entire absorbent core or no more than the width of an absorbent layer. Alternatively, the pledget may be placed anywhere within the absorbent structure. The pledget 86 remains in its pre-use condition, centrally located within the article, and is exposed to the body facing layer as the first and second portions 88 and 90 of the first absorbent layer 82 are separated. See FIG: 5B.

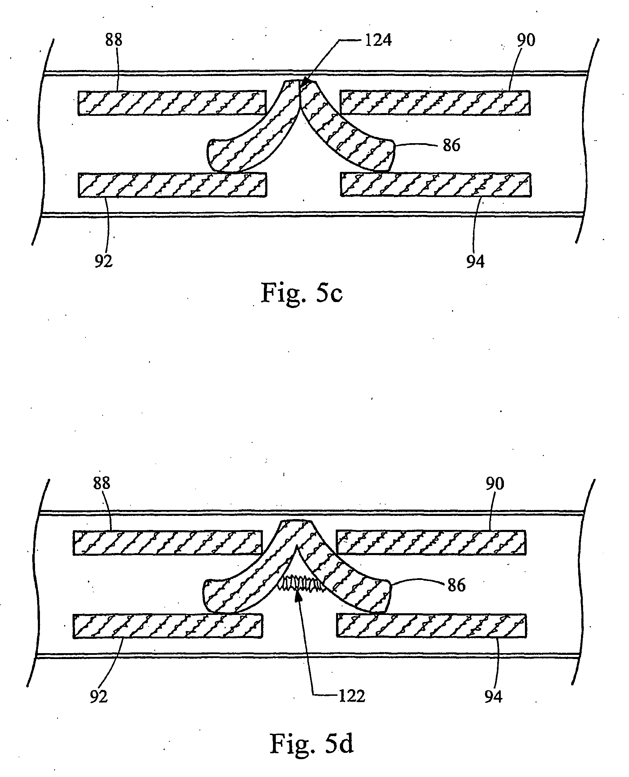

As shown in FIG. 5C, the pledget 86 may protrude upwards, slightly, toward the surface of the body facing layer 14 when the absorbent article 12 is in an in-use condition. The pledget 86 would allow for intimate body fit with the wearer's anatomy and facilitates improved fluid acquisition and distribution of that fluid to the garment facing layer 16 of the absorbent article 12. In approximately the center 124 of the pledget 86, the pledget 86 may be weakened or embossed, causing retractive forces associated with the relaxation of the product's extensible material to concentrate on the center area located between first and second portions 88 and 90. The retractive forces, or the compressive forces of the user's thighs, push the article 12 inward and catch the pledget in a protruding configuration. In the alternative, elastic material 122 may be attached to the pledget 86, contracting the pledget 86 inward, as shown in FIG. 5D.

For this embodiment, materials suitable for use in'the absorbent core 80 include airlaid layers, coform, wetlaid layers, hydroentangled pulp, tissue, foam, and meltblown microfiber material. Such absorbent materials may include superabsorbent particles or fibers. Particularly desirably would be combinations of the above materials with surge materials described previously in this invention. In such executions, the absorbent materials would be ideally suited for the absorbent pledget and the optional second absorbent layer while the surge materials wold be well-suited for the first absorbent layer.

The first portion 88 and 92 and second portion 90 and 94 of the first 82 and second 84 layers may be affixed to the opposed end portions 20 and 22 of the body facing 14 and garment facing 16 layers with hotmelt and stretchable adhesives, heat sealing, ultrasonics, embossing or by any other suitable processes or adhesives known to those in the art (not shown).

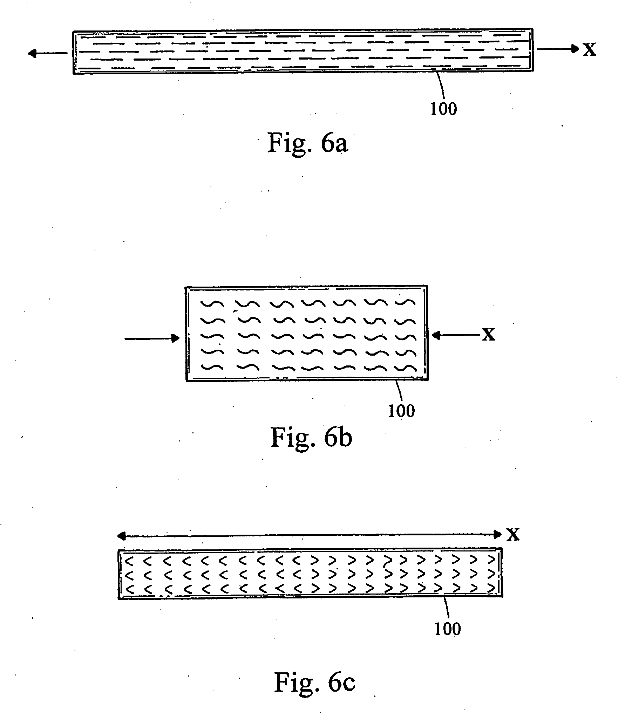

The following embodiments, shown in FIGS 6A-8C, include a material having a responsive wet-thickness attribute that will be dependent on the initial absorbent thickness and enable body waste capture and retention tailored for individual consumer needs. Materials providing a responsive wet-thickness attribute are described in U.S. Patent No. 6,238,379 to Keuhn et.al. and U.S. Patent Application No. 20030087574 to Latimer et. al.

As shown in FIGS. 6A, 6B, and 6C, an article with extensible body facing and/or garment facing layers, as described above, may be combined with an extensible absorbent core 100 with a resilient component. An example of a suitable material is described in U.S. Patent No. 6,572,735 to Wallajapet et al., "Wet-formed composite defining latent voids and macro-cavities". The resilient component can be developed based on the composition of the materials itself or can also be achieved by treating a suitable material. Examples of other suitable resilient materials in are described in U.S Patent Application No. 20030033584. In a first pre-use condition the core 100 is in a thin, stretched state, See FIG. 6A. The consumer at the point of usage allows the absorbent core 100 to increase in thickness to a second pre-use condition as the core 100 relaxes due to the resiliency of material, FIG. 6B. The user may then customize the absorbent core 100 by applying tension to the core 100. The core 100 may be maintained in a desired state or in-use condition as shown in FIG. 6C, by use of by heat sealing, ultrasonics, embossing, or by any other suitable process or adhesive known in the art. In one embodiment, the absorbent core may be affixed to the garment and/or body facing layer with fastening tabs (not shown). When the garment facing layer is extended, the absorbent core is also expanded to the thickness and bulk desired by the user.

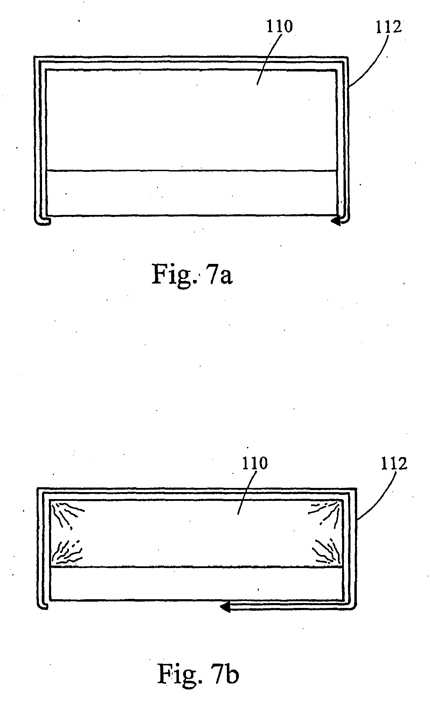

As shown in FIGS. 7A and 7B, another embodiment of the present invention includes an article with extensible body facing layer and/or an extensible garment facing layer, as described above, and an absorbent core 110 that may be tailored to a desired thickness and stabilized to maintain that thickness during use by stretching and refastening an adjustable shell 112 surrounding the absorbent core 110. FIG. 7A shows this embodiment in a pre-use condition. The resilient absorbent core 110 is surrounded by an adjustable shell 112. The user may then adjust the thickness or bulk of the absorbent core 110 by reducing or compressing the circumference of the adjustable shell 112 and stabilizing the absorbent material, FIG. 7B.

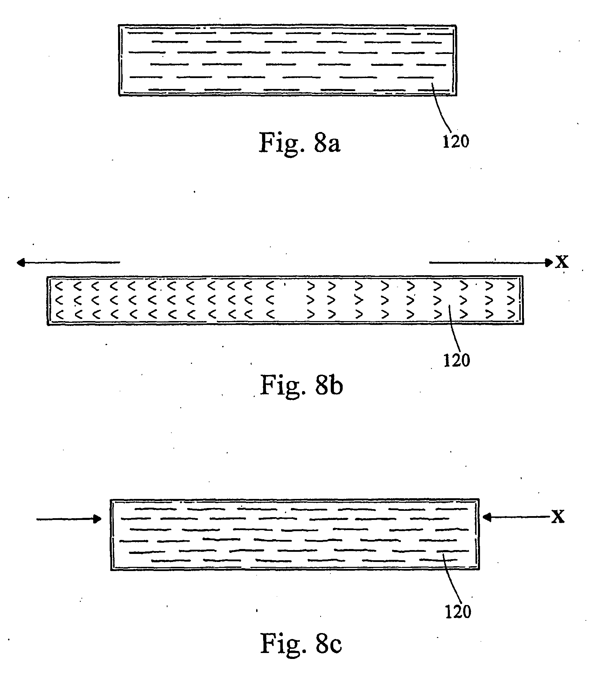

In yet another embodiment of the present invention, an article with extensible body facing and garment facing layers, as described above, is combined with an absorbent core 120 including stretch activatable multicomponent fibers and films, as shown in FIGS. 8A, 8B, and 8C. The core 120 is packaged in a relaxed, pre-use condition (FIG. 8A). The thickness of the core 120 may be increased due to interspatial instability between the components of polymer interface by the application of tension moving the core 120 to a second pre-use condition (FIG. 8B). Suitable examples of materials that comprised of stretch-activatable films are described in U.S. Patent Application Serial Number 10/647,414, "Absorbent article formed with microlayered films." Also, suitable examples of stretch-activatable fibers that are suitable for this product are described in U.S. Patent Application Serial Number 10/232,059. In the case of these materials, the consumer can adjust the thickness of the absorbent core 120 to an in-use condition by controlling the degree of stretch and using a tab or other suitable attachment components which may be attached to the absorbent core. The user is then able to select a desired thickness level as shown in FIG. 8C.

Although the present invention has been described with reference to preferred embodiments, those skilled in the art will recognize that changes may be made in form and detail without departing from the spirit and scope of the invention. As such, it is intended that the foregoing detailed description be regarded as illustrative rather than limited and that it is the appended claims, including all equivalents thereof, which are intended to define the scope of the invention.

## Claims

1. A customizable absorbent article comprising:
an extensible body facing layer having a first end and a second end spaced along a longitudinal axis, wherein said body facing layer is extensible along said longitudinal axis;
an extensible garment facing layer having a first end and a second end spaced along said longitudinal axis, wherein said garment facing layer is extensible along said longitudinal axis; and
an absorbent core disposed between said body facing layer and said garment facing layer, wherein said absorbent core is affixed to at least one of said body facing and said garment facing layer at at least one location between said first and second ends;
wherein said absorbent core further comprises an absorbent pledget having a length and a first absorbent layer;
said first absorbent layer having a first portion and a second portion, said first and second portions being movable relative to each other from a pre-use condition, wherein said first portion and said second portion are approximately adjacent one another, to a post use condition, wherein said first portion and said second portion are separated from one another by a distance which is equal to or less than said length of said pledget; and
wherein a portion of said pledget is exposed to at least one of said body facing layer and said garment facing layer when said first and second portions of said absorbent layer are in said post-use condition.

2. The customizable absorbent article of claim 1, wherein said article further comprises a second absorbent layer having a first portion and a second portion, said first and second portions being movable relative to each other from a pre-use condition, wherein said first portion and said second portions are approximately adjacent one another, to a post-use condition, wherein said first portion and said second portion are separated from one another by a second distance equal to or less than said length of said pledget.

3. The customizable absorbent article of claim 1 or 2, wherein said pledget protrudes toward a surface of the body facing layer when the article is in said post-use condition.

4. The customizable absorbent article of claim 3, wherein the center of the pledget is weakened or embossed.

5. The customizable absorbent article of claim 3, further comprising an elastic material attached to said pledget to contract the pledget inward.

6. A customizable absorbent article comprising:
an extensible body facing layer having a first end and a second end spaced along a longitudinal axis, wherein said body facing layer is extensible along said longitudinal axis;
an extensible garment facing layer having a first end and a second end spaced along said longitudinal axis, wherein said garment facing layer is extensible along said longitudinal axis;
an absorbent core that is substantially permanently deformable, said absorbent core being manually movable from a pre-use condition, wherein said absorbent core has a first thickness, to an in-use condition, wherein said absorbent core has a second thickness that is different than said thickness of said absorbent core in said pre-use condition; and
wherein said absorbent core is minimally affixed to at least one of said body facing layer and said garment facing layer.

7. The customizable absorbent article of claim 6, wherein said first thickness of said absorbent core is greater than said second thickness.

8. The customizable absorbent article of claim 6, wherein said first thickness of said absorbent core is less than said second thickness.

9. A customizable absorbent article comprising:
a cover layer movable between a pre-use condition and an in-use condition; and
a resilient inner core movable between a pre-use condition and an in-use condition, wherein said inner core has a first thickness when in said pre-use condition and wherein said inner core has a second thickness when in said in-use condition;
said inner core being movable from said pre-use condition to said in-use condition by moving said cover layer from said pre-use condition to said in-use condition.

10. The customizable absorbent article of claim 9, wherein said first thickness is greater than said second thickness.

11. The customizable absorbent article of claim 9, wherein said first thickness is less than said second thickness.

12. The customizable absorbent article of claim 9, wherein said resilient inner core further comprises a second pre-use condition, wherein said inner core has a third thickness.
